# EUROPEAN PATENT APPLICATION

(11) **EP 3 981 356 A1**
(43) Date of publication of application: **13.04.2022**
(21) Application number: 21201695.0
(22) Date of filing: 08.10.2021
(51) Int. Cl.: A61B 90/40, A61B 90/00, A61C 17/08

(54) **DENTAL SUCTION MANIFOLD**

(30) Priority: 09.10.2020 IT 202000023800
(71) Applicant: Cerboni, Fabio, 58022 Follonica, Grosseto (IT)
(72) Inventor: Cerboni, Fabio, 58022 Follonica, Grosseto (IT)
(74) Representative: Leihkauf, Steffen Falk

(57) **Abstract**

The present invention relates to a dental handpiece with accessory (supplementary) suction of aerosols capable of improving the operating safety of the dentist, or of other staff close to the patient, and of the patient himself/herself, against the risk of transmission of a virus among them.

In particular, the invention relates to a suction handpiece (1) configured for use in dentistry, comprising a handpiece body (2) having a distal end (2a) and a proximal end (2b), and a suction bell (3) arranged at said distal end (2a), the handpiece body (2) comprising a saliva suction cannula (7) arranged coaxially and protruding from the suction bell (3), a compressed air supply channel (11) and one or more outlets connected to the suction bell (3), wherein the saliva suction cannula (7), the channel (11) and the outlets are in flow communication with a connection cavity (20) placed at the proximal end (2b) of the handpiece body (2) and connected to a suction pipe (4).

## Description

### Field of the invention

The present invention relates to a dental handpiece with accessory suction of aerosols capable of improving the operating safety of the dentist, or of other staff close to the patient, and of the patient himself/herself, against the risk of transmission of a virus among them.

### Background art

The work of the dentist and support staff requires operating directly on the patient's teeth or in his/her mouth using tools such as a specific dental turbine or micromotor drill, an ultrasonic scaler device, a bicarbonate spray dispenser, a water-air spray gun.

The aforesaid tools are generally indicated with the term "handpiece" since they have an elongated grip, or handpiece body, and an operating head, adapted to work on the patient, arranged at a distal end of the handpiece body.

Among the various types of known handpieces, some of which have been summarily listed above, there is a cannula-holder suction handpiece comprising a handpiece body which is grippable by the operator, and a suction cannula having a proximal end connected to a suction duct which passes through the handpiece body and leads to a suction and collection unit, and a free distal end which is adapted to be positioned at a minimum distance from the work area in the patient's mouth so as to suck and remove excess saliva along with the working products which form in such a work area.

In all these cases, the handpiece is arranged, in use, close to the teeth or an area of the oral cavity to be worked on.

The action of the high speed of the elements which work on the teeth, the vibrations produced in the oral cavity, or the action of sprays in the oral cavity, produce a high quantity of microscopic-size solid or liquid particles which are formed in the oral cavity and spread in the air from the oral cavity, in the form of aerosol.

This aerosol of air and particles dispersed therein is extremely dangerous due to the risk of transmission of viruses or bacteria from the patient to the dentist or assistant, as such pathogens, if present, are normally incorporated in such aerosol.

This risk is particularly felt in recent times, due to the spread of the virus referred to as Covid-19.

The need is therefore felt to remove the aforesaid aerosol with operating safety as soon as it exits the patient's mouth, to prevent such aerosol from spreading in the air and from reaching the dentist and staff close to the patient.

The technique is known of sanitizing the environment around the dental unit by means of a portable sanitization machine capable of sucking the air contained in the room and filtering it continuously, and then introducing it purified into the same room.

However, this prior art is not without disadvantages.

In fact, even if such a sanitization machine is capable of sucking the air contained in the room and sanitizing it, is not capable of intercepting and removing the aerosol directly near the patient's mouth before such aerosol hits the dentist or staff close to the patient, risking to infect him.

In other words, the aforesaid machinery is not capable of protecting the dentist, or the staff close to the patient, from the risk of contagion with any virus or bacterium of the patient's airways.

Therefore, suggesting a device which is capable of minimizing the risk of contagion of the dentist or staff present close to the patient and which is simultaneously not bulky and of low cost is a particularly felt issue.

### Summary of the invention

It is the object of the present invention to provide a device for sucking the aerosol emitted from the oral cavity of a patient undergoing dental or orthodontic treatment which allows to satisfy the aforesaid requirements and at least partially obviate the drawbacks mentioned above with reference to the prior art.

These and further objects are achieved by a suction handpiece in accordance with the independent claim.

In particular, the present invention relates to a suction handpiece configured for use in dentistry, comprising a handpiece body having a distal end and a proximal end and a suction bell arranged at said distal end, the handpiece body comprising a saliva suction cannula arranged coaxially and protruding from the suction bell, a compressed air supply channel and one or more outlets connected to the suction bell, in which the saliva suction cannula, the channel and the outlets are in flow communication with a connection cavity placed at the proximal end of the handpiece body and connected to a suction pipe.

Further objects, solutions, and advantages are present in the embodiments described below and claimed in the dependent claims.

### Brief description of the drawings

The invention will be shown below by describing some embodiments thereof, given by way of non-limiting example, with reference to the accompanying drawings, in which:
- figure 1 shows a diagrammatic perspective view of a suction handpiece according to the invention;
- figure 2 shows a detail of the handpiece in figure 1;
- figure 3 shows a perspective view of the handpiece in figure 1 from a different point of view;
- figure 4 shows a plan view according to the direction A of the handpiece in figure 3;
- figure 5 shows a sectional view of a detail of the handpiece according to the invention;
- figure 6 shows a sectional view of a detail of the handpiece according to an embodiment.

### Description of the preferred embodiments

With reference to the figures, a suction handpiece according to the invention is globally denoted with reference numeral 1. The handpiece 1 comprises a handpiece body 2 having a distal end 2a and a proximal end 2b and a suction bell 3 arranged at the distal end 2a of the body 2.

The proximal end 2b of the body 2 is connected to a pipe 4, in turn connected to suction means 5, typically a suction pump, by means of the interposition of valve means 6, typically a membrane solenoid valve.

The handpiece body 2 coaxially comprises a saliva suction cannula 7 protruding from the suction bell 3. The saliva suction cannula 7, in the example shown in the figures, is fixed in a coaxial position by means of centering means 8, made for example by means of a collar 10 - in which the saliva suction cannula 7 passes - from which rods 9 radially depart (three in the figures) which are fixed to the walls or to the inner edge of the suction bell 3.

A compressed air supply channel 11 comprising a distal end 11a and a proximal end 11b is associated with the handpiece body 2.

The proximal end 11b of the channel 11 is connected to a compressed air delivery pipe 12, in turn connected, by means of the interposition of valve means 13, typically a solenoid valve, to a compressed air source 14, such as the compressor used by the so-called "dental unit".

The distal end 11a of the compressed air supply channel 11 opens into a cavity 15 which acts as a compensating reservoir for the compressed air and from which one or more sleeves 15a depart, extending parallel to the channel 11 and ending with a substantially conical nozzle 16 having a dispensing opening 16a facing the proximal end 2b of the handpiece body 2. The assembly formed by the channel 11, the cavity 15 and each of the sleeves 15a has a U-shaped configuration which allows the compressed air, which is introduced into the cavity 15 in the direction of the distal end 2a of the handpiece body 2, to outflow in the opposite direction.

The nozzle 16 is arranged in a funnel-shaped element 18 comprising a tube 19 which opens into a cavity 20 of the handpiece body 2 with the suction pipe 4.

The suction bell 3 is connected to one or more outlets 17 which open into the respective funnel-shaped elements 18.

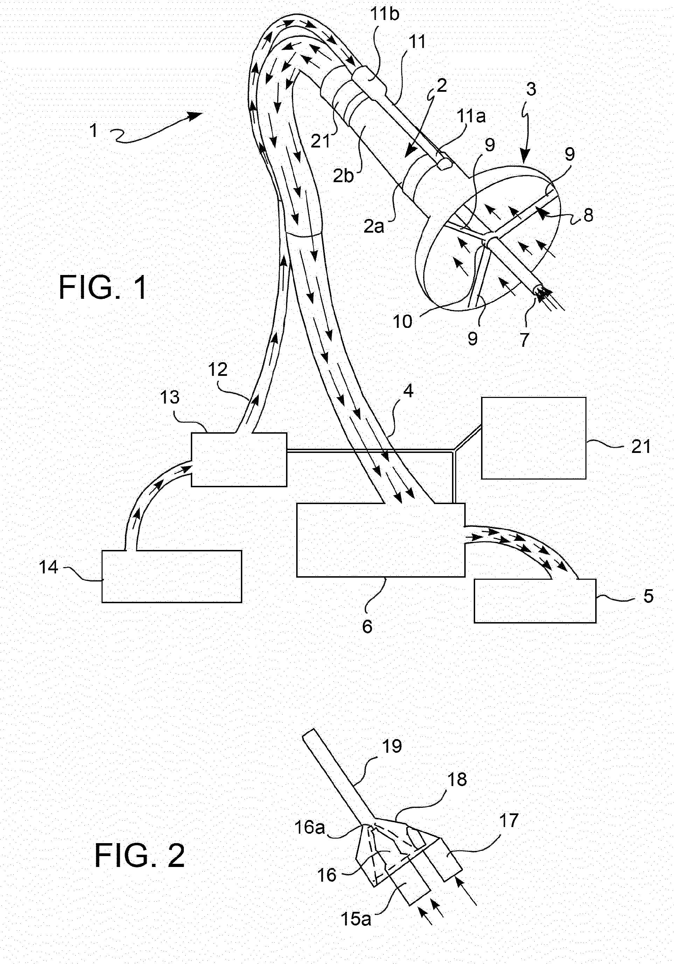

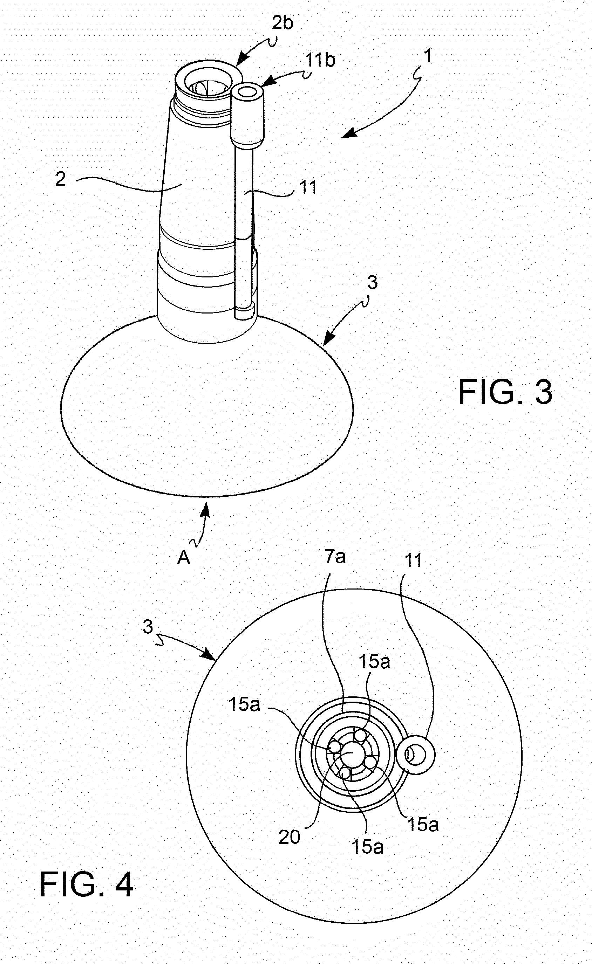

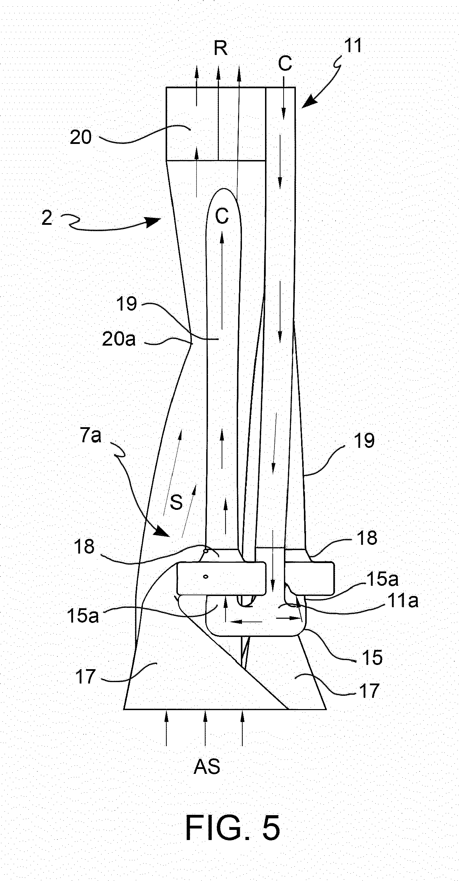

As shown in figures 4 and 5, the saliva suction cannula 7 (in fig. 1) opens inside the body 2 into a suction chamber 7a for the saliva which gathers in the connection cavity 20 with the aerosol sucked by the suction bell 3.

There is a calibrated restriction 20a between the suction chamber 7a and the connection cavity 20. The ducts 19 enter the connection cavity 20 of the handpiece body 2 downstream of said calibrated restriction 20a so as to avoid turbulence and, by exploiting Bernoulli's law, accelerate the outflow, increasing the general efficiency of the system.

The suction bell 3 and the saliva suction cannula 7 are configured to suck, respectively, the aerosol generated in the patient's oral cavity and the saliva. The fluid thus sucked by means of the suction means 5 then gathers in the pipe 4.

Figure 5 clearly shows the flows of fluids which pass through the channels inside the handpiece body 2. C indicates the entering compressed air flow, AS indicates the aerosol sucked by means of the suction bell 3, S indicates the saliva sucked by means of the cannula 7 and R indicates the combined flow of saliva and aerosol which is sent into the suction pipe 4.

The compressed air, introduced into the funnel-shaped element 18 by means of the nozzle 16, causes the suction of the aerosol by the Venturi effect, making such suction very efficient.

The suction bell 3 can be removed from the handpiece body 2, so as to facilitate the sanitization thereof or, in the case of a disposable bell, to allow the replacement thereof.

As mentioned, the compressed air source 14 can be a compressor or a cylinder of compressed air.

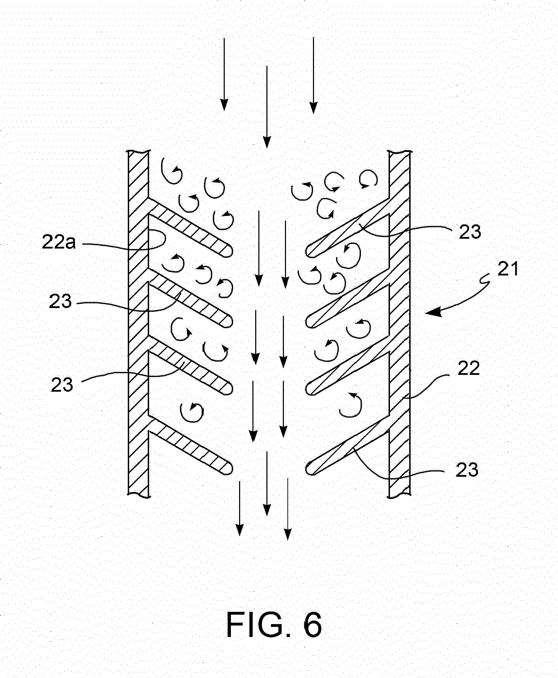

As shown in figures 1 and 6, in certain embodiments the handpiece of the invention comprises a silencer element 21. In fact, it has been noted that, under certain operating conditions, the air flow causes an annoying noise.

As shown in figure 6, the silencer element 21 comprises a tubular body 22 inside which a plurality of conical rings 23 is arranged in series, departing from the inner surface 22a of the tubular body 22 and protruding towards the center of the tubular body. 22 with the flare facing the flow of sucked air/aerosol (indicated by the arrows in figure 6). Thereby, the air/aerosol intercepted by the conical rings changes the flow from laminar to turbulent and the noise is thus reduced.

In certain embodiments, the handpiece 1 according to the invention comprises a command and control unit 21 which controls:
- the opening and closing of the valve means 6, 13 which put in flow communication the suction pipe 4 with the suction means 5 and the compressed air delivery pipe 12 with the compressed air source 14, respectively;
- the activation of the suction means 5 and the compressed air source 14.

To this end, the command and control unit 21 can be operatively connected to a proximity sensor or a contact button which sends an activation signal to the command and control unit 21 when the handpiece 1 is detached from the tool holder on the dentist's console.

The handpiece 1 according to the invention allows one or more of the suggested advantages to be achieved, since it has a small size, a low invasiveness for the patient and does not require a separate tool as compared to the saliva suction handpiece normally used by dentists.

In order to meet contingent needs, those skilled in the art can make changes and adaptations to the above-described embodiments of the device or can replace elements with others which are functionally equivalent without departing from the scope of the following claims. All the features described above as belonging to a possible embodiment may be implemented irrespective of the other described embodiments.

Moreover, the drawings are not necessarily to scale.

All the features described herein (comprising any claim, abstract, and drawings) and/or in any step of the method described herein may be combined in any combination, except for the combinations in which at least some of such features and/or steps are mutually exclusive.

## Claims

1. A suction handpiece (1) configured for use in dentistry, comprising a handpiece body (2) having a distal end (2a) and a proximal end (2b), and a suction bell (3) arranged at said distal end (2a), the handpiece body (2) comprising a saliva-suction cannula (7) arranged coaxially and protruding from the suction bell (3), a compressed air supply channel (11) and one or more outlets (17) connected to the suction bell (3), wherein the saliva-suction cannula (7), the channel (11) and the outlets (17) are in flow communication with a connection cavity (20) placed at the proximal end (2b) of the handpiece body (2) and connected to a suction pipe (4).

2. A suction handpiece (1) according to claim 1, wherein the suction pipe (4) is connected to suction means (5) by means of the interposition of valve means (6) .

3. A suction handpiece (1) according to claim 1 or 2, wherein the compressed air supply channel (11) comprises a distal end (11a) and a proximal end (11b), the proximal end (11b) of the channel (11) being connected to a compressed air delivery pipe (12), in turn connected to a compressed air source (14) by means of the interposition of valve means (13).

4. A suction handpiece (1) according to any one of claims 1 to 3, wherein the distal end (11a) of the compressed air supply channel (11) opens into a cavity (15) which acts as a compensating reservoir for the compressed air and from which one or more sleeves (15a) depart, extending parallel to the channel (11) and ending with a substantially conical nozzle (16) having a dispensing opening (16a) facing the proximal end (2b) of the handpiece body (2).

5. A suction handpiece (1) according to claim 4, wherein each nozzle (16) is arranged in a funnel-like element (18) comprising a tube (19) which opens into said connection cavity (20) of the handpiece body (2) and wherein said one or more outlets (17) open into the respective funnel-like elements (18).

6. A suction handpiece (1) according to claim 4 or 5, wherein the assembly formed by the channel (11), the cavity (15) and each of the sleeves (15a) has a U-shaped configuration which allows the compressed air, which is introduced into the cavity (15) in the direction of the distal end (2a) of the handpiece body (2), to outflow in the opposite direction.

7. A suction handpiece (1) according to any one of claims 1 to 6, wherein the saliva-suction cannula (7) opens inside the handpiece body (2) into a suction chamber (7a) for the saliva which gathers in the connection cavity (20) with the aerosol sucked by the suction bell (3).

8. A suction handpiece (1) according to claim 7, wherein between said suction chamber (7a) and said connection cavity (20) there is a calibrated restriction (20a), and wherein the ducts (19) enter into the connection cavity (20) of the handpiece body (2) downstream of said calibrated restriction (20a).

9. A suction handpiece (1) according to any one of claims 1 to 8, wherein the suction bell (3) is removable from the handpiece body (2).

10. A suction handpiece (1) according to any one of claims 3 to 9, comprising a command and control unit (21) which controls:
- the opening and closing of the valve means (6, 13) which put in flow communication the suction pipe (4) with the suction means (5) and the compressed air delivery pipe (12) with the compressed air source (14), respectively;
- the activation of the suction means (5) and the compressed air source (14).

11. A suction handpiece (1) according to claim 10, wherein the command and control unit (21) is operatively connected to a proximity sensor or a contact button which sends an activation signal to the command and control unit (21) when the handpiece (1) is detached from the tool holder on the dentist's console.

12. A suction handpiece (1) according to any one of claims 1 to 11, comprising a silencer element (21) which comprises a tubular body (22) inside which a plurality of conical rings (23) is arranged in series, departing from
the inner surface (22a) of the tubular body (22) and protruding towards the center of the tubular body (22)
with a flare facing the flow of sucked air/aerosol.
